# EUROPEAN PATENT APPLICATION

(11) **EP 3 185 207 A1**
(43) Date of publication of application: **28.06.2017**
(21) Application number: 15833368.2
(22) Date of filing: 07.08.2015
(51) Int. Cl.: G06T 5/00, A61B 1/04, H04N 1/407

(54) **IMAGE PROCESSING DEVICE, IMAGE PROCESSING METHOD, AND IMAGE PROCESSING PROGRAM**

(30) Priority: 22.08.2014 JP 2014169409
(71) Applicant: OLYMPUS CORPORATION, Hachioji-shi Tokyo 192-8507 (JP)
(72) Inventor: MATSUURA, Hiroto, Tokyo 151-0072 (JP)
(74) Representative: Gunzelmann, Rainer
(86) International application number: PCT/JP2015/072449
(87) International publication number: WO 2016/027693

(57) **Abstract**

A high-quality image in which glare caused by high-luminance bright spots is suppressed is obtained, while maintaining the sense of sharpness and resolution of the image as a whole. Provided is an image processing apparatus (1) including a high-luminance-pixel identifying unit (2) that identifies pixels having luminance values equal to or greater than a prescribed threshold in an input image; a frequency-distribution creating unit (3) that creates a frequency distribution of the luminance values of all of the pixels identified by the high-luminance-pixel identifying unit (2); a storage unit (4) that stores a target frequency distribution; and a luminance correcting unit (5) that corrects the luminance values of the pixels identified by the high-luminance-pixel identifying unit (2) so that the frequency distribution created by the frequency-distribution creating unit (3) approaches the target frequency distribution stored in the storage unit (4).

## Description

### {Technical Field}

The present invention relates to an image processing apparatus, an image processing method, and an image processing program.

### {Background Art}

In the related art, with the aim of improving, in an image, the contrast representation of a portion that should be displayed at high luminance, there is a known technique in which high-luminance gamma correction is performed on a high-luminance region or a region in which the amount of change in luminance is large, in order to further increase the luminance, and in which basic gamma correction is performed on regions other than these regions (for example, see Patent Literature 1).

### {Citation List}

### {Patent Literature}

{PTL 1}
Japanese Unexamined Patent Application, Publication No. 2010-38954

### {Summary of Invention}

### {Technical Problem}

However, in cases such as those in which illumination light is reflected at indentations and protrusions on blood vessels or tissue, and many small high-luminance bright spots exist, such as, for example, endoscope images in which the inner wall of the body cavity is acquired, with the technique in Patent Literature 1, the glare caused by the high-luminance bright spots is not improved, and the quality of the endoscope image decreases.

The present invention has been conceived in light of the above circumstances, and an object thereof is to provide an image processing apparatus, an image processing method, and an image processing program with which it is possible to obtain a high-quality image in which glare due to high-luminance bright spots is suppressed, while maintaining the sense of sharpness and resolution of the image as a whole.

### {Solution to Problem}

In order to realize the above object, the present invention provides the following solutions.

An aspect of the present invention provides: an image processing apparatus comprising: a high-luminance-pixel identifying unit that identifies pixels having luminance values equal to or greater than a prescribed threshold in an input image; a frequency-distribution creating unit that creates a frequency distribution of the luminance values of all of the pixels identified by the high-luminance-pixel identifying unit; a storage unit that stores a target frequency distribution; and a luminance correcting unit that corrects the luminance values of the pixels identified by the high-luminance-pixel identifying unit so that the frequency distribution created by the frequency-distribution creating unit approaches the target frequency distribution stored in the storage unit.

With this aspect, pixels having luminance values equal to or greater than a prescribed threshold are identified from the input image by the high-luminance-pixel identifying unit, and a frequency distribution of the luminance values of all of the identified pixels is created by the frequency-distribution creating unit. Then, with the luminance correcting unit, the created frequency distribution and the target frequency distribution stored in the storage unit are compared, and the luminance values of the pixels identified by the high-luminance-pixel identifying unit are corrected so that the created frequency distribution approaches the target frequency distribution.

It is possible to make the frequency distribution of the luminance values of the identified high-luminance pixels approach the target frequency distribution, and it is possible to obtain a high-quality image in which glare due do high-luminance points is suppressed, while maintaining the sense of sharpness and resolution of the image as a whole.

In the above-described aspect, the luminance correcting unit may perform gamma correction on the basis of the frequency distribution created by the frequency-distribution creating unit.

In the above-described aspect, the luminance correcting unit may perform gamma correction on the basis of the gradient of the frequency distribution created by the frequency-distribution creating unit.

Another aspect of the present invention provides an image processing method comprising: a high-luminance-pixel identifying step of identifying pixels having a luminance value equal to or greater than a prescribed threshold in an input image; a frequency-distribution creating step of creating a frequency distribution of the luminance values of all of the pixels identified in the high-luminance-pixel identifying step; and a luminance correcting step of correcting the luminance values of the pixels identified in the high-luminance-pixel identifying step so that the frequency distribution created in the frequency-distribution creating step approaches a target frequency distribution.

In the above-described aspect, in the luminance correcting step, gamma correction may be performed on the basis of the frequency distribution created in the frequency-distribution creating step.

In the above-described aspect, in the luminance correcting step, gamma correction may be performed on the basis of the gradient of the frequency distribution created in the frequency-distribution creating step.

Another aspect of the present invention provides an image processing program that enables a computer to execute: a high-luminance-pixel identifying step of identifying pixels having a luminance value equal to or greater than a prescribed threshold in an input image; a frequency-distribution creating step of creating a frequency distribution of the luminance values of all of the pixels identified in the high-luminance-pixel identifying step; and a luminance correcting step of correcting the luminance values of the pixels identified in the high-luminance-pixel identifying step so that the frequency distribution created in the frequency-distribution creating step approaches a target frequency distribution.

### {Advantageous Effects of Invention}

The present invention affords an advantage in that it is possible to obtain a high-quality image in which glare due to high-luminance bright spots is suppressed, while maintaining the sense of sharpness and resolution of the image as a whole.

### {Brief Description of Drawings}

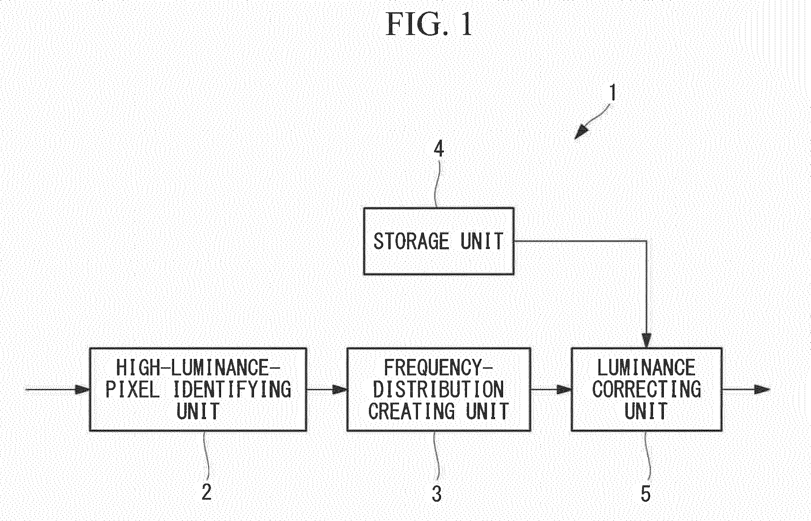

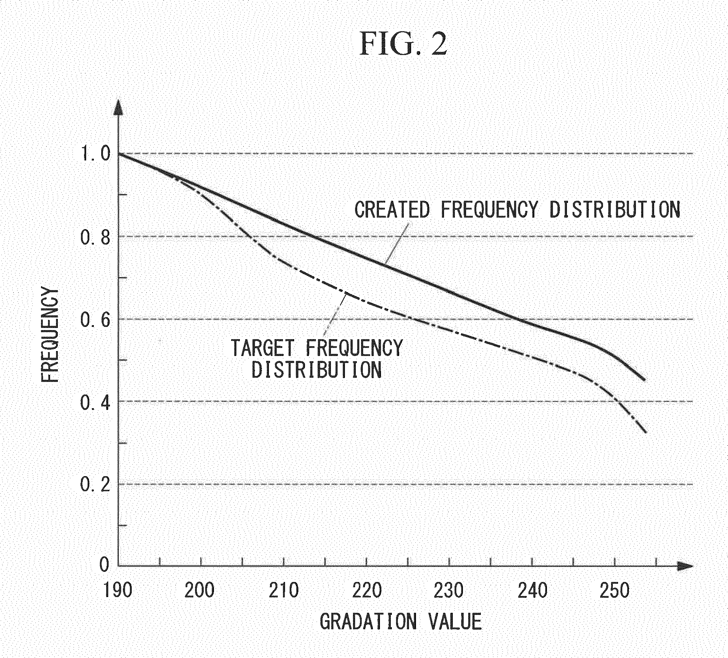

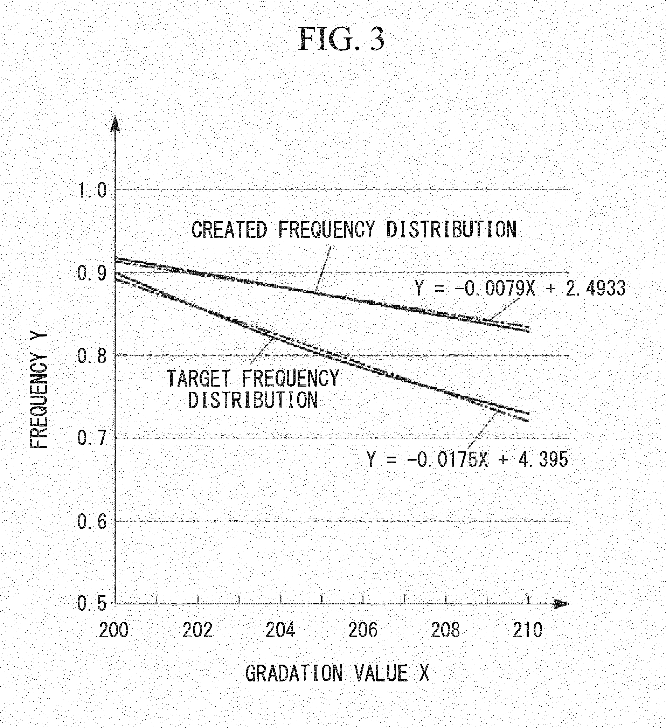

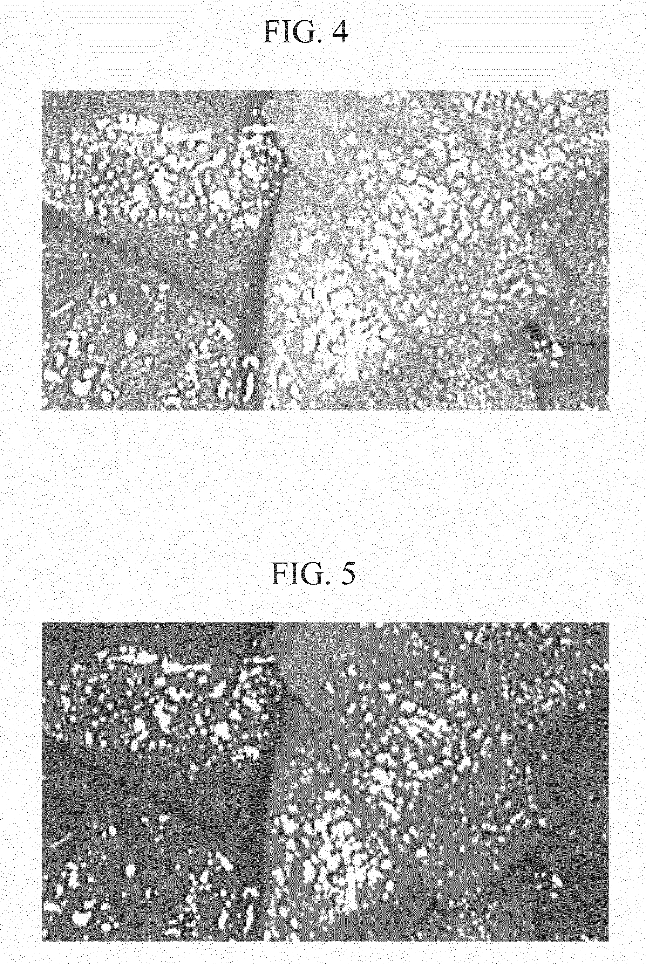

{Fig. 1} Fig. 1 is a diagram showing the overall configuration of an image processing apparatus according to an embodiment of the present invention.
{Fig. 2} Fig. 2 is a graph showing a frequency distribution created by the frequency-distribution creating unit in the image processing apparatus in Fig. 1 and a target frequency distribution stored in a storage unit.
{Fig. 3} Fig. 3 is a graph showing a state in which the frequency distribution in Fig. 2 is approximated by a straight line.
{Fig. 4} Fig. 4 is a diagram showing an endoscope image before image processing.
{Fig. 5} Fig. 5 is a diagram showing an endoscope image after image processing by the image processing apparatus in Fig. 1.

### {Description of Embodiment}

An image processing apparatus and an image processing method according to an embodiment of the present invention will be described below with reference to the drawings.

An image processing apparatus 1 according to this embodiment is applied to processing of an image obtained by an endoscope or the like and, as shown in Fig. 1, includes: a high-luminance-pixel identifying unit 2 that identifies, in an input image, pixels having a luminance value equal to or greater than a prescribed threshold; a frequency-distribution creating unit 3 that creates a frequency distribution of the luminance values of all of the identified pixels; a storage unit 4 that stores a target frequency distribution; and a luminance correcting unit 5 that corrects luminance values of the pixels identified by the high-luminance-pixel identifying unit 2 so that the frequency distribution created by the frequency-distribution creating unit 3 approaches the target frequency distribution stored in the storage unit 4.

The high-luminance-pixel identifying unit 2 compares the input image with a prescribed threshold to identify pixels having a luminance value greater than or equal to the threshold. In this embodiment, for example, an image signal in which the luminance is represented by 8 bits is input, and in the high-luminance-pixel identifying unit 2, by binarizing the image with a gradation value of 190 serving as the threshold, pixels having a high luminance greater than or equal to a gradation value of 190 are extracted.

For all pixels identified in the high-luminance-pixel identifying unit 2, the frequency-distribution creating unit 3 counts the numbers of pixels having a luminance value equal or greater than the threshold while gradually varying the threshold and stores the numbers in association with the thresholds. Accordingly, it is possible to obtain a frequency distribution of the luminance values of high-luminance pixels. An example of the frequency distribution created by the frequency-distribution creating unit 3 is shown by the solid line in Fig. 2. Fig. 2 is a frequency distribution in which the frequencies of the luminances are normalized to the frequency at a gradation value of 190.

The storage unit 4 stores a target frequency distribution, as shown by the broken line in Fig. 2, for example. This frequency distribution is a frequency distribution of an image considered to be a low-glare, high-quality vivid image by processing an image obtained from the same observed scene.

The luminance correcting unit 5 corrects the coefficient of gamma correction to be applied to the image so that the frequency distribution created by the frequency-distribution creating unit 3 approaches the target frequency distribution stored in the storage unit 4. More specifically, as shown in Fig. 3, only the luminance band that influences the amount of glare is approximated by a straight line. Here, as an example, the 200-210 nm range is approximated by a straight line.

As a result, the gradient of the approximating straight line (broken line) in the above wavelength range of the created frequency distribution (solid line) was -0.0079, whereas the gradient of the approximating straight line (broken line) for the target frequency distribution (solid line) became -0.0175.

Thus, the luminance correcting unit 5 is configured to set the gamma correction coefficient so that the gradient of the approximating straight line of the created frequency distribution approaches the gradient of the approximating straight line of the target frequency distribution.

In other words, the luminance correcting unit 5 compares the gradient of the approximating straight line of the created frequency distribution and the gradient of the approximating straight line of the target frequency distribution and calculates an adjustment amount for the gamma correction coefficient according to the difference therebetween.

In other words, when the difference between the gradients of the approximating straight lines is large, by correcting the gamma curve so that the change in the gamma correction coefficient in the high-luminance band becomes larger, a clear difference can be provided between the frequency distribution at a wavelength of 200 nm and the frequency distribution at a wavelength of 210 nm, and by means of the luminance difference, it is possible to correct the gamma curve to produce a vivid image. In the example shown in Fig. 3, before correction, because the difference between the frequency distribution at the wavelength of 200 nm and the frequency distribution at the wavelength of 210 nm is small, the image is not vivid and has the impression that white bright points are spread throughout the entire screen.

An image processing method used in the thus-configured image processing apparatus 1 according to this embodiment will be described below.

To perform image processing using the image processing apparatus 1 according to this embodiment, the acquired image is input to the high-luminance-pixel identifying unit 2, and pixels having gradation values of 190 and higher are extracted (high-luminance-pixel identifying step). Next, the extracted high-luminance pixels are input to the frequency-distribution creating unit 3, and a frequency distribution for each gradation value is created (frequency-distribution creating step).

In the case of an endoscope image, as shown in Fig. 4, illumination light emitted from the endoscope onto indentations and projections on blood vessels or tissue is reflected, causing small, high-luminance bright spots, and thus, a low-quality endoscope image is obtained. The frequency distribution of such an endoscope image is represented by the solid line in Fig. 2.

The created frequency distribution is sent to the luminance correcting unit 5. The target frequency distribution stored in the storage unit 4 is also input to the luminance correcting unit 5.

The luminance correcting unit 5 compares the frequency distribution created by the frequency-distribution creating unit 3 and the target frequency distribution stored in the storage unit 4, and sets the gamma correction coefficient so that the created frequency distribution approaches the target frequency distribution. Then, the input endoscope image is processed in accordance with the set gamma correction coefficient (luminance correcting step). Accordingly, it is possible to obtain a corrected image by image processing, as shown in Fig. 5.

This corrected endoscope image is a high-quality image having few high-luminance bright spots and no glare. In other words, with the image processing apparatus and the image processing method according to this embodiment, high-luminance bright spots do not disappear, but frequency changes in the high-luminance region are large, while high-luminance bright spots remain at about the same level; that is to say, an advantage is afforded in that, it is possible to produce a vivid final image by reducing the fraction of the number of glare points having the same luminance in the high-luminance region.

In this embodiment, although high-luminance pixels in the entire input image are counted, instead of this, a specific region in the image may be specified, high-luminance pixels in the specified region may be counted, and the coefficient for gamma correction to be applied only to that region may be set, and processing carried out.

In this embodiment, although the luminance correcting unit determines the gamma correction coefficient according to the gradient of the frequency distribution created by the frequency-distribution creating unit, it may determine the gamma correction coefficient according to the frequency distribution itself.

In addition, the image processing method according to this embodiment can also be realized in the form of an image processing program that can be executed on a computer.

### {Reference Signs List}

- 1: image processing apparatus
- 2: high-luminance-pixel identifying unit
- 3: frequency-distribution creating unit
- 4: storage unit
- 5: luminance correcting unit

## Claims

1. An image processing apparatus comprising:
a high-luminance-pixel identifying unit that identifies pixels having luminance values equal to or greater than a prescribed threshold in an input image;
a frequency-distribution creating unit that creates a frequency distribution of the luminance values of all of the pixels identified by the high-luminance-pixel identifying unit;
a storage unit that stores a target frequency distribution; and
a luminance correcting unit that corrects the luminance values of the pixels identified by the high-luminance-pixel identifying unit so that the frequency distribution created by the frequency-distribution creating unit approaches the target frequency distribution stored in the storage unit.

2. An image processing apparatus according to claim 1, wherein the luminance correcting unit performs gamma correction on the basis of the frequency distribution created by the frequency-distribution creating unit.

3. An image processing apparatus according to claim 2, wherein the luminance correcting unit performs gamma correction on the basis of the gradient of the frequency distribution created by the frequency-distribution creating unit.

4. An image processing method comprising:
a high-luminance-pixel identifying step of identifying pixels having a luminance value equal to or greater than a prescribed threshold in an input image;
a frequency-distribution creating step of creating a frequency distribution of the luminance values of all of the pixels identified in the high-luminance-pixel identifying step; and
a luminance correcting step of correcting the luminance values of the pixels identified in the high-luminance-pixel identifying step so that the frequency distribution created in the frequency-distribution creating step approaches a target frequency distribution.

5. An image processing method according to claim 4, wherein in the luminance correcting step, gamma correction is performed on the basis of the frequency distribution created in the frequency-distribution creating step.

6. An image processing method according to claim 5, wherein in the luminance correcting step, gamma correction is performed on the basis of the gradient of the frequency distribution created in the frequency-distribution creating step.

7. An image processing program that enables a computer to execute:
a high-luminance-pixel identifying step of identifying pixels having a luminance value equal to or greater than a prescribed threshold in an input image;
a frequency-distribution creating step of creating a frequency distribution of the luminance values of all of the pixels identified in the high-luminance-pixel identifying step; and
a luminance correcting step of correcting the luminance values of the pixels identified in the high-luminance-pixel identifying step so that the frequency distribution created in the frequency-distribution creating step approaches a target frequency distribution.
